# EUROPÄISCHE PATENTANMELDUNG

(11) **EP 1 428 544 A2**
(43) Veröffentlichungstag der Anmeldung: **16.06.2004**
(21) Anmeldenummer: 04006726.6
(22) Anmeldetag: 23.02.1999
(51) Int. Cl.: A61M 16/04

(54) **Trachealbeatmungsvorrichtung**

(30) Priorität: 09.03.1998 DE 29804142 U; 15.06.1998 DE 19826547; 02.10.1998 DE 19845415
(62) Teilanmeldung aus: 99939165.9
(71) Anmelder: Dr. Fred Göbel Patentverwaltung GmbH, 69115 Heidelberg (DE)
(72) Erfinder: Göbel, Fred G., Dr., 69115 Heidelberg (DE)
(74) Vertreter: Grünecker, Kinkeldey, Stockmair & Schwanhäusser Anwaltssozietät

(57) **Zusammenfassung**

Die Erfindung bezieht sich auf eine Trachealbeatmungsvorrichtung, insbesondere Trachealtubus oder Trachealkanüle, die die Trachea zum Beatmen eines Patienten möglichst dicht verschließt, mit einer die Trachea unterhalb der Glottis blockierenden Cuffblase, durch die eine Beatmungskanüle hindurchgeführt ist, wobei die Cuffblase in gefülltem, ohne Begrenzung frei auffaltbarem Zustand größer ist als in gefülltem Zustand in der Trachea platziert, und aus flexiblen Weichfolienmaterial besteht. Um mit dieser Vorrichtung einen Patienten möglichst schonend bei niedrigen Drücken über lange Zeit mit geringem Infektionsrisiko zu intubieren, wird erfindungsgemäß vorgeschlagen, dass die Cuffblase mit Faltenwurf an der Trachea anliegt und derart ausgebildet ist, dass die am toten Ende einer Falte entstehende Faltenöse einen geringen Durchmesser aufweist, der den freien Durchfluss des Sekrets durch Faltenöse hemmend entgegenwirkt.

## Beschreibung

Die Erfindung bezieht sich auf eine Trachealbeatmungsvorrichtung, insbesondere Trachealtubus oder Trachealkanüle, die die Trachea zum Beatmen eines Patienten möglichst dicht verschließt, mit einer die Trachea unterhalb der Glottis blockierenden Cuffblase, durch die eine Beatmungskanüle hindurchgeführt ist, wobei die Cuffblase in gefülltem, ohne Begrenzung frei auffaltbarem Zustand größer ist als in gefülltem Zustand in der Trachea platziert, und aus flexiblem Weichfolienmaterial besteht.

Bei einem aus der DE 196 38 935 bekannten Trachealtubus dieser Gattung wird vorgeschlagen, für die Cuffblase ein folienartiges, äußerst dehnbares Material zu verwenden, das sich eng an die Trachea bzw. an die Binnenstrukturen des subglottischen Kehlkopfes (Larynx) anschmiegt. Um die Tamponade des subglottischen Raumes zu optimieren, wird empfohlen, die Cuffblase der Morphologie des auszufüllenden Raumes entsprechend zu präformieren. Damit soll unerwünschter Faltenwurf vermieden werden. Dies soll gewährleisten, dass die Folie eng an der Trachea anliegt, so dass möglichst kein Sekret aus dem Rachenraum in die Lunge gelangt. Mikroaspiration von Sekret über die Cuffblase hinweg wird damit erheblich reduziert.

Sekret, das in das distale tracheabronchiale System gelangt, ist für die Entstehung des überwiegenden Teils aller beatmungsassoziierter Pneumonien (Lungenentzündungen verantwortlich.

Bei dem in der DE 196 38 935 vorgeschlagenen Trachealtubus dehnt sich die Cuffblase beim Aufblasen elastisch auf und legt sich ohne Faltenwurf eng an die Wand der Trachea an. Übersteigt der Fülldruck der Cuffblase den Durchblutungsdruck der Schleimhaut versorgenden Gefäßbettes, so können schwerwiegende strukturelle Läsionen des Epithels die Folge sein. Vor allem bei langzeitintubierten Patienten sollte der Fülldruck in der Cuffblase auf einem möglichst niedrigen, die Schleimhautperfusion nicht beeinträchtigenden Niveau gehalten werden. Wird der Fülldruck hingegen zu niedrig gewählt, kann dies zur Leckage bakteriell besiedelten Rachensekrets über die Cuffblase hinweg und damit zur Besiedelung und Infektion des Lungengewebes führen.

Für die Langzeitintubation wurde daher vorgeschlagen, Cuffblasen zu verwenden, die sich bei mäßigem Fülldruck in der Trachea entfalten, ohne dass dabei der Cuffmantel selbst aufgedehnt werden muss. Der Durchmesser der frei entfalteten Cuffblase ist dabei größer als der der zu verschließenden Trachea. Der residuale Anteil des Cuffmantels stülpt sich bei der trachealen Blockung der Cuffmanschette in Falten. Andererseits hat sich aber herausgestellt, dass derartige Cuff-Manschetten eine hohe Durchlässigkeit für Rachensekret aufweisen, was eine erhöhte Pneumoniegefahr bedeutet. Die Sekretleckage dieser Cuffblasen liegt im Bereich von Millilitern pro Sekunde und entspricht damit quantitativ sogar einer Makroaspiration.

Es ist daher davon auszugehen, dass konventionelle Cuffblasen für den Großteil der bei langzeitbeatmeten Patienten häufigen Pneumonien (Inzidenz 10% bis 80%, abhängig vom jeweiligen Patientengut) verantwortlich ist.

Der Erfindung liegt die Aufgabe zugrunde, einen Trachealtubus der eingangs genannten Gattung dahingehend zu verbessern, dass ein Patient damit möglichst schonend bei niedrigen Drücken über lange Zeit intubiert werden kann, wobei das Infektionsrisiko gering sein soll.

Diese Aufgabe wird erfindungsgemäß gelöst mit einem gattungsgemäßen Trachealtubus, der dadurch gekennzeichnet ist, dass die Cuffblase mit Faltenwurf an der Trachea anliegt und derart ausgebildet ist, dass die am toten Ende einer Falte entstehende Faltenöse einen geringen Durchmesser aufweist, der dem freien Durchfluss des Sekrets durch die Faltenöse hemmend entgegenwirkt.

Das Dichtungsvermögen der Cuff-Manschette lässt sich so wesentlich verbessern, das Risiko einer beatmungsassoziierenden Pneumonie ist entsprechend vermindert.

Erstaunlicherweise lässt sich der Sekretfluss durch die spezifische Ausbildung der Cuff-Faltung im Bereich der Faltenöse, also am Faltengrund beeinflussen. Während im Stand der Technik bisher davon ausgegangen wurde, dass Cuffblasen mit Faltenwurf wegen des geringen Füll-Druckes nicht ausreichend dicht an der Trachea anliegen können, gibt die Erfindung einen Weg zur Hemmung des Sekret-Flusses, der im Bereich der Faltenöse einer Falte ansetzt. Wenn am Faltengrund der Durchmesser der Faltenöse ausreichend gering ist, wird der Durchfluss des Sekrets durch die Faltenöse gehemmt. Beim Blocken der Cuff-Manschette können die resultierenden Faltenösen am tiefen Ende der Falte z.B. durch Auswahl des Materials oder der Foliendicke in ihrem Durchmesser derart reduziert werden, dass der Sekretfluss gebremst wird oder im Idealfall zum Stillstand kommt.

Dies ist überraschend, da die Leckage stets als Problem des Drucks aufgefasst wurde. Eine konstruktive Änderung des Faltenwurfes war bisher noch nie in Betracht gezogen worden.

Vorzugsweise ist die Faltenöse in Kapillargröße ausgebildet. Es resultieren dann ausreichende Adhäsionskräfte des Sekretes an der Faltenöse bzw. ein ausreichender viskositätsabhängiger Widerstand des Sekretes, um den Sekretfluss zu reduzieren. Die Strömungsgeschwindigkeit in der kapillargroßen Faltenöse ist dann geringer als die theoretisch mögliche Geschwindigkeit ohne Adhäsions- bzw. Viskositiätskräfte, so dass über die Zeit weniger Sekret hindurchfließt. Im Optimalfall kann der Durchmesser der Öse so klein gestaltet sein, dass der Sekretfluss ganz zum Erliegen kommt.

Besonders vorteilhaft kann der Durchmesser der Faltenöse weniger als 0,11 oder 0,05 mm betragen. Unter 0, 1 mm setzt bereits eine gewisse Hemmung der Fließgeschwindigkeit des Sekrets durch die Öse ein. Bei einem Faltenösendurchmesser von unter 0,05 mm verlangsamt sich der Sekretfluss weiter und kommt annähernd zum Erliegen.

Günstigerweise kann die Wandstärke des Folienmaterials derart dünn gewählt sein, dass sich der Innenradius der sich bildenden Faltenösen bei physiologisch verträglichen Fülldrucken soweit reduziert, dass Sekret an einem freien Durchfluss gehindert wird. Je flexibler und dünner das Material, desto geringer ist der Durchmesser der Öse.

Die Wandstärken konventioneller Cuffblasen liegen überwiegend im Bereich von 0,06 bis 0, 1 mm.

Es wird vorgeschlagen, dass die Wandstärke der Folie etwa kleiner oder gleich 0,02 mm beträgt. Besteht die Cuffblase aus einer derartigen Folie, setzt bei regulärem Fülldruck bereits eine Hemmung des Sekretflusses durch die Faltenöse ein.

Als Variante der Erfindung beträgt die Wanddicke der Folie etwa 0,01 bis 0,005 mm. Im Bereich einer Wandstärke von 0,01 bis 0,005 mm kann mit einer weichen, flexiblen Folie bereits eine befriedigende Hemmung des Sekretflusses bzw. seine Stase im Bereich des Faltengrundes erreicht werden.

Folien momentan verfügbarer Materialien mit Wandstärken von unter 0,005 mm sind für die erfindungsgemäße Cuffblasengestaltung nicht ausreichend reißfest. Sollten geeignete, ausreichend resistente Materialien verfügbar sein, sind Foliendicken unter 0,005 mm zur Erreichung optimaler Dichtungseigenschaften anzustreben.

Gemäß einer bevorzugten Ausführungsform kann das Folienmaterial dar Cuffblase zum Beispiel aus Polyethlenteraphtalat (PETP), niedrig dichtem Polyethylen (LDPE), Polyvinylchlorid (PCV) oder Polyurethan (PU) bestehen. Diese Materialien sind körperverträglich und eignen sich in entsprechend dünnwandiger Verarbeitung besonders gut zur Ausbildung eines dicht schließenden Faltenwurfes. Copolymerbeimischungen zur Modifikation der Materialeigenschaften sind denkbar (z.B. LDPE-EVA).

Möglicherweise besteht die Cuffblase aus einem sich selbst anhaftendem Material, dessen Adhäsion zur Reduktion des lichten Durchmessers einer Öse am Faltengrund beiträgt.

Als Variante der Erfindung kann die Wanddicke der Folie im Bereich des Faltenwurfes dünner sein, als in dem, der Trachealschleimhaut unmittelbar anliegenden, faltenfreien Bereich. Im dünnwandigeren Cuffbereich bilden sich bevorzugt Falten aus, da die Folie hier leichter verformbar ist. Der Faltengrund kann aufgrund der geringeren Wandstärke Ösen kleineren Durchmessers ausbilden. Im dickwandigeren Cuffbereich zwischen den Falten verhält sich der Cuffmantel etwas starrer, so dass er nur gerundet an der Wand der Trachea anliegt.

In besonderer Weise können sich die in eine Falte gegenüberliegenden Faltenwände im Bereich nahe des Faltengrundes miteinander verbunden sein. Die Verbindungsstelle kann unmittelbar benachbart zu der sich bildenden Faltenöse vorgesehen sein, so dass durch sie die Größe der Faltenöse auf einen gewünschten Durchmesser einzustellen ist.

Vorzugsweise können die sich gegenüberliegenden Faltenwände am toten Ende der Falte, die Faltenöse ausfüllend, miteinander verbunden sein und so den Sekretfluss sicher unterbinden.

Denkbar können die sich gegenüberliegenden Faltenwände einer Falte miteinander verschweißt oder verklebt sein.

In besonderer Weise kann sich in der Falte, an den Verbindungsbereich der sich gegenüberliegenden Faltenwände angrenzend, ein im Querschnitt in der Faltentiefe variabler Faltenabschnitt anschließen, in dem die sich gegenüberliegenden Faltenwände nicht stofflich miteinander verbunden sind. Über einen derartigen präformierten Faltenwurf mit variabler Faltentiefe kann sich die Cuffblase der Trachea, dem Konzept residualen Cuffmantels, in Größe und Form anpassen.

Bei einem aus DE 196 38 935 bekannten Trachealtubus dieser Gattung wird vorgeschlagen, die konventionelle Cuffmanschette des Trachealtubus durch eine zweite, sich unmittelbar an die Cuffmanschette nach oral anschließende, und den sog. subglottischen Raum (Raum zwischen Cuffmanschettenoberrand und den Stimmlippen) vollständig ausfüllende Tamponierblase zu ergänzen. Diese besteht aus einem folienartigen, äußerst dehnbaren Material, das sich unter Aufdehnung eng an die Binnenstrukturen des subglottischen Raumes anschmiegt. Um die Tamponade des subglottischen Raumes zu optimieren, wird empfohlen die Blase der Morphologie des auszufüllenden Raumes entsprechend zu präformieren. Durch eine möglichst glatte, faltenfreie Oberfläche der Tamponierblase soll jede Ansammlung von Sekreten bzw. die Ausbildung eines subglottischen Erregerreservoirs verhindert werden.

Das Aufdehnen eines derartigen Verdrängungskörpers geht jedoch vor allem im Bereich des morphologisch aufwendig gestalteten inneren Larynx (Kehlkopfes) mit der Entstehung von Druckspitzen im Bereich prominenter, in den Binnenraum hineinreichender Strukturen einher.

Übersteigt der Fülldruck der Tamponierblase den Perfusionsdruck des schleimhautversorgenden Gefäßbettes, können vor allem im Bereich des dorsolateralen subglottischen Kehlkopfes schwerwiegende Läsionen der Wandstrukturen die Folge sein.

Um einer Druckschädigung des Kehlkopfes vorzubeugen, wird empfohlen die Tamponierblase ebenfalls residualvolumig zu gestalten, d.h. ihr Volumen bei freier Entfaltung soll das Volumen des auszufüllenden inneren Kehlkopfes überschreiten. Die Tamponierblase entspricht dabei den erfindungsgemäßen Prinzipien zur dichtenden und schonenden Cuffblasengestaltung. Der Ausbildung der beschriebenen kapillarartigen Strukturen wird so vorgebeugt.

Da bei vielen Anwendungen eine zuverlässige, mechanisch belastbare Verankerung des Trachealtubus in der Luftröhre gefordert wird, darf abhängig von der jeweiligen Materialqualität eine gewisse Mindestwandstärke der fixierenden Cuffmanschette nicht unterschritten werden. Der Ausbildung flüssigkeitsdrainierender Faltenösen kann so, trotz weitgehender Reduzierung der Wandstärke, nicht in allen Fällen ausreichend vorgebeugt werden.

Um dennoch ein optimales Dichtungsverhalten zu gewährleisten, schlägt die Erfindung die Ergänzung der mechanisch fixierenden Cuffmanschette durch eine zusätzliche dichtende, den zuvor beschriebenen Gestaltungsprinzipien zur Dichtung und Gewebeverträglichkeit entsprechende, Tamponierblase vor. Die Tamponierblase kann mit einem minimalen Fülldruck von vorzugsweise 10 bis 15 mbar beaufschlagt sein, welcher lediglich das Entfalten der dünnen, dichtenden Blasenwandung zur Aufgabe hat.

Die Tamponierblase kann hinsichtlich Anordnung und Bezug zum Tubusschaft bzw. zur fixierender Cuffmanschette den in DE 196 38 935 beschriebenen Ausführungsformen entsprechen.

Es wird vorgeschlagen, dass die fixierende Cuffmanschette an der kaudalen Seite der Vorrichtung, und die Tamponierblase relativ dazu kranial angebracht ist. Die Fixier-Cuffmanschette wird bei der Intubation über den Ringknorpel des Kehlkopfes hinweg, bis vorzugsweise in den Bereich des mittleren trachealen Drittels vorgeschoben, wo sie sicher und tracheal verträglich verankert wird. Die kranial dazu angeordnete Tamponierblase kann sich in Richtung des subglottischen Raumes ausdehnen und sorgt dort, der fixierenden Cuffmanschette vorgeschaltet, für eine Abdichtung gegen vom Rachen her einsickerndes Sekret.

Denkbar können die Fixiercuffmanschette und die Tamponierblase in sequenzieller Anordnung auf der Beatmungskanüle positioniert sein. Die Tamponierblase kann in ihrer Ausdehnung nach kranial den sog. subglottischen Raum teilweise erfassen, bis zur Glottisebene oder geringfügig darüber hinaus reichen. Da beide Blasen über entsprechende, innerhalb des Tubusschaftes angebrachte Versorgungslumina separat befüllt werden, können die Funktionen von Fixier-Cuffmanschette und Tamponierblase bei serieller Anordnung weitgehend unabhängig voneinander kontrolliert werden.

Vorzugsweise ist die Verbindungsstelle zwischen den beiden seriell angeordneten Blasen derart ausgestaltet, dass sich in ihrem Bereich, im gefüllten, tracheal entfalteten Zustand, kein Sekret ansammeln kann.

Günstigerweise kann die Fixier-Cuffmanschette mindestens bereichsweise, vorzugsweise vollständig, von der Tamponierblase umschlossen sein. Die äußere Tamponierblase kann sich also nach kaudal bis in einen variablen Bereich der Fixier-Cuffmanschette ausdehnen. Die Bildung eines Erregerreservoirs zwischen den Blasen wird so verhindert.

In einer bevorzugten Variante ist die den Tubus tracheal fixierende Cuffmanschette von der Tamponierblase völlig umschlossen. Die Tamponierblase reicht vom kaudalen Ende über das kraniale Ende der Cuffmanschette bis in den sog. subglottischen Raum hinein bzw. bis in den Bereich der Stimmlippenebene oder geringfügig darüber hinaus. Bei dieser ineinandergeschachtelten Ausführungsform der Kombination einer fixierenden Cuffmanschette mit einer Tamponierblase schlägt die Erfindung eine besondere Art der Handhabung vor.

Nach konventioneller Intubation soll initial die äußere flüssigkeitsdicht schließende Tamponierblase befüllt werden und sich der Wandung des auszufüllenden Binnenraums unter minimalem Druck anschmiegen. Anschließend wird dann die im Inneren angeordnete Fixiercuffmanschette zur Stabilisierung des Tubus in der Trachea in gewohnter Weise und mit üblichen Fülldrücken entfaltet. Die Fixiercuffmanschette hat somit keinen Flüssigkeitskontakt, die eventuelle Ausbildung von Faltenösen im Mantel des inneren Cuffs also keinen flüssigkeitsdrainierenden Effekt.

Um ein gegenseitiges Anhaften der beiden Blasen bei ihrer Entfaltung zu verhindern und ihr unabhängiges mechanisches Verhalten bei der Beatmung zu gewährleisten, wird vorgeschlagen ein geringe Menge eines trennendes Mediums, wie z.B. Öl oder Talkum, in den Raum zwischen den Blasen einzubringen.

Wird die mit minimalem, gewebeschonendem Fülldruck beaufschlagte Tamponierblase bis in den Bereich der Glottis bzw. geringfügig darüber hinaus ausgedehnt, wird der potentielle Eintrittsweg für keimhaltiges Sekret maximal verlängert. Das Sekretvolumen wird durch die verdrängende Tamponierblase auf einen schmalen, den epitheleigenen Abwehrfaktoren exponierten Film reduziert und somit in seiner Fließgeschwindigkeit maximal reduziert. Insgesamt wird die Effizienz der lokalen Abwehrmechanismen dadurch wesentlich optimiert.

Da die Stase erregerhaltigen Materials oberhalb der tracheal fixierenden Cuffmanschette praktisch vollständig unterbunden wird, kann chronisch entzündlich bedingten Veränderungen der Schleimhaut zudem vorgebeugt werden.

Reicht die Tamponierblase über die Stimmlippen hinaus bis in den supraglottischen Bereich, kann der permanente traumatisierende Kontakt des Tubusschaftes mit den Stimmlippen durch die spannungsfreie Umkleidung der Stimmlippen mit dem Mantel der Tamponierblase reduziert werden.

Auch die Dichtigkeit jedes herkömmlichen Tubus (high-volume/low-pressure, high-pressure/low-volume Cuff oder intermediär gestalteter Cuff), welcher nicht den zuvor beschriebenen Vorzug der Elimination des subglottischen Erregerreservoirs bietet, kann durch die ineinandergeschachtelte Anordnung des fixierten Cuffs und einer nur wenige Mikrometer wandstarken Tamponierblase hinsichtlich Dichtigkeit und Gewebeverträglichkeit optimiert werden. Die äußere Tamponierblase sollt die Fixiermanschette dabei in ihrer kranialen und kaudalen Ausdehnung nur geringfügig überschreiten bzw. in ihren Abmessungen der Fixiermanschette entsprechen. Beide Blasen sind getrennt befüllbar. Auch hier soll durch die initiale Entfaltung der äußeren, wenige Mikrometer dünnen, dicht schließenden Hülle verhindert werden, dass die Ausbildung flüssigkeitsleitender Tubuli der inneren wandstärkeren Cuffmanschette zur Ursache einer Leckage von Sekreten werden kann.

Eine derartige Anordnung von dichtender und stabilisierender Hülle macht die Anwendung der Erfindung nicht nur auf Trachealtuben, sondern in besonderer Weise auch auf Trachealkanülen denkbar. Trachealkanülen werden nicht über den Kehlkopf, sondern über eine operativ angelegte Öffnung (Stoma) in die Luftöhre eingeführt.

Die Aufrechterhaltung des Fülldruckes in sämtlichen beschriebenen Cuff- und Tamponierblasen wird durch extrakorporal angebrachte Reservoirs gewährleistet. Diese können, entsprechend dem Lanz'schen Prinzip, mit einem selbstregulierenden Ventilmechanismus ausgestattet, oder in der Art simpler, ventiltragender Reservoirblasen gestaltet sein.

Zur Abschätzung des gewünschten Fülldrucks kann eine aufgedruckte Figur bzw. eine spezifische Form der Reservoirblase gewählt werden, welche sich bei entsprechendem Füllzustand in spezifischer Weise verändert.

Um zu vermeiden, dass Druckschwankungen innerhalb der Trachea bzw. des Larynx zu einer Aufdehnung der Wandstrukturen führen, sollte die Materialcompliance der Reservoirblase die der Cuff- bzw. Tamponierblase überschreiten.

Der zur Tamponierblase führende Versorgungsschenkel soll ausreichend großlumig gewählt werden, um einen raschen Druckausgleich herstellen zu können.

Zur Befüllung der dichtenden bzw. fixierenden Cuffblasen kann jedes geeignete Fluid verwendet werden.

Bei Verwendung von Flüssigkelten kann zudem auf einen Ventilmechanismus verzichtet und die Befüllung alleine über eine offene Flüssigkeitssäule reguliert werden.

Die erfindungsgemäße Dichtung des bei der Intubation entstehenden trachealen bzw. laryngealen Restlumens durch die Reduktion der Wandstärke des Cuffmantels bis in den Bereich weniger Mikrometer macht auch die flüssigkeitsdichte Tamponade der intubierten Neugeboren-, Säuglings- und Kleinkindertrachea denkbar.

Wegen der hohen geweblichen Verletzlichkeit gegenüber konventionell gestalteten Cuffblasen wird bei deren Intubation bislang auf jede dichtende Vorrichtung verzichtet. Durch einen langstreckigen, die Trachea und den Larynx ausfüllenden, mit minimalsten Drücken (vorzugsweise 5 mbar) beaufschlagten Tamponierballon, wäre eine geweblich verträgliche, flüssigkeits- und gasdichte Abdichtung der äußerst empfindlichen oberen Luftwege möglich.

Ausführungsbeispiele der Erfindung sind in den Zeichnungen dargestellt und werden nachstehend erläutert. Es zeigen:
- Fig. 1: einen Längsschnitt durch die Wand einer Trachea mit einem platzierten Trachealtubus gemäß einer ersten Ausführungsform der Erfindung,
- Fig.2: eine Querschnittsansicht längs der Schnittlinie II-II in Figur 1,
- Fig.3: eine vergrößerte Darstellung der Einzelheit III von Figur II,
- Fig.4: eine vergrößerte Darstellung der Einzelheit IV von Figur II,
- Fig.5: einen erfindungsgemäßen Trachealtubus gemäß einer zweiten Ausführungsform in einem Frontalschnitt durch einen Kehlkopf mit angrenzenden anatomischen Strukturen,
- Fig.6: einen erfindungsgemäßen Trachealtubus gemäß einer dritten Ausführungsform,
- Fig.7: einen Ausgleichsballon für einen erfindungsgemäßen Trachealtubus, nur in teilweise gefülltem Zustand,
- Fig.8: den Ausgleichsballon von Figur 7 in optimal gefülltem Zustand,
- Fig.9: eine erfindungsgemäße Trachealkanüle gemäß einer vierten Ausführungsform,
- Fig. 10: einen erfindungsgemäßen Trachealtubus gemäß einer fünften Ausführungsform und
- Fig. 11: eine erfindungsgemäße Trachealbeatmungsvorrichtung gemäß einer sechsten Ausführungsform.

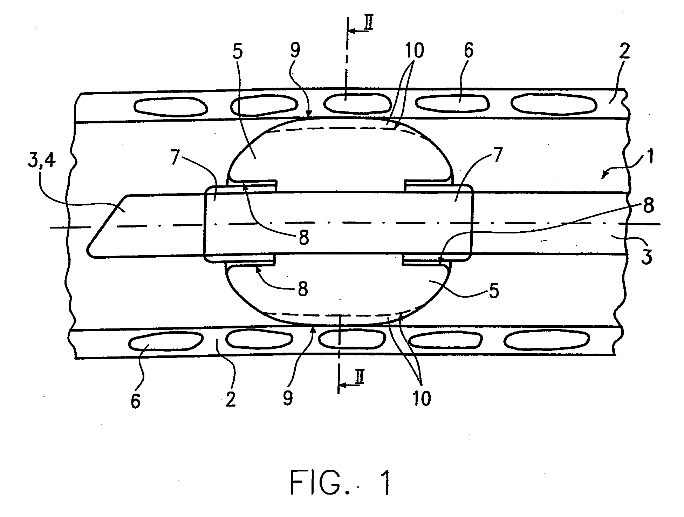

In Figur 1 ist eine erste Ausführungsform eines erfindungsgemäßen Trachealtubus 1 bzw. einer Trachealbeatmungsvorrichtung dargestellt, der in einer Trachea 2 platziert ist. Eine hohle Beatmungskanüle 3 endet an ihrem kaudalen Ende 4 schrägwinklig zu ihrer Längsachse. An dem kaudalen Ende 4 tritt Beatmungsluft in die Lunge ein und aus. Die Beatmungskanüle 3 führt in nicht dargestellte Weise über den Kehlkopf und den Rachenraum aus dem Mund des Patienten heraus und ist dort mit geeigneten Beatmungsgeräten (nicht dargestellt) verbunden.

An der Beatmungskanüle 3 ist eine Cuffblase 5 nahe dem kaudalen Ende 4 angebracht. Durch die Cuffblase hindurch erstreckt sich die Beatmungskanüle. Der Tubus 1 ist derart in die Trachea 2 eingeführt, dass die Cuffmanschette 5 im Bereich der mittleren Trachea zu liegen kommt. Von der Trachea sind andeutungsweise ringförmige Trachealspangen 6 dargestellt.

Die Cuffblase 5 ist etwa ballonartig ausgebildet und umgibt die die Beatmungskanüle 3 etwa schlauchartig. Sie ist an zwei beabstandet voneinander vorgesehenen Enden 7 an der Beatmungskanüle 3 befestigt, z. B. durch Bandagieren, Aufschrumpfen, Schweißen oder Kleben, so dass das befestigte Ende 7 der Blase 5 fluiddicht an der Kanüle 3 anliegt.

Die Kanüle 3 ist über eine in Figur 1 nicht dargestellte Verbindung mit Fluid mit einem gewünschten Druck befüllbar.

Im Bereich der befestigten Enden 7 ist an der Blase eine Umschlagsfalte 8 vorgesehen, die sich ringförmig um die Beatmungskanüle 3 erstreckt. Mit Hilfe der Umschlagsfalte 8 sind ausreichende axiale Bewegungen von Cuffblase 5 und Beatmungskanüle 3 relativ zueinander möglich, ohne dass die Anlagefläche der Cuffblase 5 an der Trachea 2 medizinisch nachteilig beeinflusst wird.

In Figur 1 ist die Cuffblase 5 in gefülltem Zustand in der Trachea platziert dargestellt. Der Fülldruck beträgt etwa 20 bis 30 mbar (vorzugsweise 25 mbar). Wäre die Cuffblase 5 nicht in der Trachea platziert, würde sie sich, vollständig gefüllt, über den trachealen Durchmesser hinaus entfalten. Bei der trachealen Platzierung liegt sie umfangseitig mit einer Auflagefläche 9 innenseitig der Trachea 2 an. Der residuale Cuffmantel stülpt sich in überwiegend radial nach innen gerichtete Falten 10.

Das Material der Cuffblase 5 besteht aus einem flexiblen Weichfolienmaterial, vorzugsweise mit einer Wanddicke von unter 0,02mm, optimal jedoch 0,01 bis 0,005 mm oder sogar unter 0,005 mm. Das Folienmaterial ist körperverträglich und besteht z.B. aus Polyethylenteraphtalat (PETP), niedrig dichtem Polyethylen (LDPE), Polyvinylchlorid (PVC) oder Polyurethan (PU).

Bei einem klinisch üblichen Fülldruck von 25 bis 30 mbar wird die Folie nicht bzw. nur minimal gedehnt, sondern flexibel gebogen. Sie liegt mit Falten 10 der Trachea 2 an. In Figur 1 sind die Faltenbereiche strichliniert angedeutet. Die Falten 10 sind typischerweise axial zur Trachea 2 und zur Beatmungskanüle 3 angeordnet. Sie sind in der Mehrzahl längs, parallel zum Tubusschaft 3 ausgerichtet. Die Wanddicke der Folie ist im Bereich der Ausbildung der Falten 10 vorzugsweise dünner als im faltenfreien Bereich der Anlagefläche 9.

Wahlweise kann der gewählte Fülldruck der Cuffblase zwischen 10 und 30 mbar liegen.

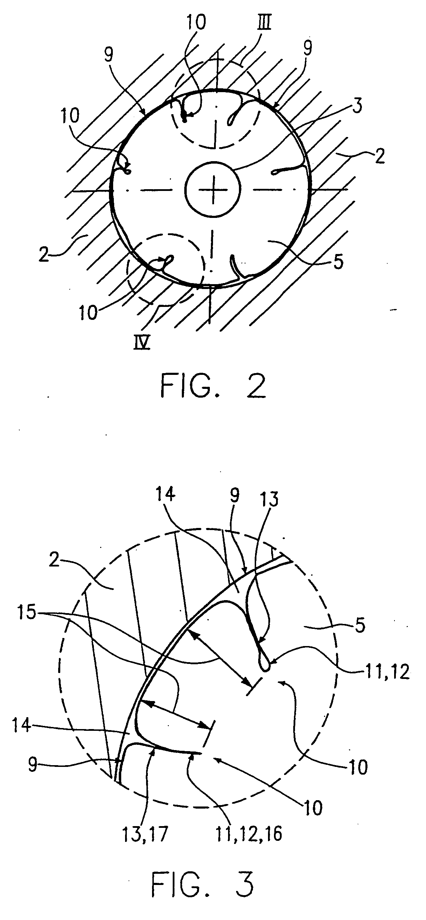

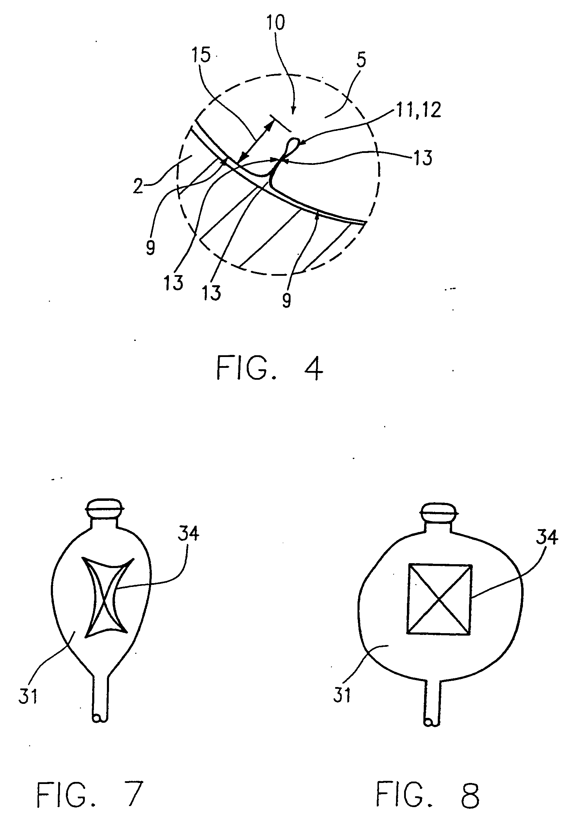

In Figur 2 ist eine Querschnittsansicht längs der Schnittlinie II-II in Figur 1 dargestellt, wobei bezüglich Teile gleicher Bezugszeichen auf die Beschreibung zu Figur 1 verwiesen wird. In Figur 2 ist ersichtlich, wie sich bei gefülltem Zustand der Cuffblase 5, etwa radial nach innen hervorstehend, die Falten 10 ausbilden. Die Faltung resultiert aus dem Durchmesser der Cuffblase 5, der in frei aufgefaltetem Zustand größer ist als der Querschnitt der auszufüllenden Trachea 2. Jede der Falten 10 hat an ihrem Ende, dem Faltengrund 11, eine Faltenöse 12, die sich durch den Umschlag des Cuffmantels in diesem Bereich ergibt. Diesbezüglich wird auf die Figuren 3 und 4 verwiesen, die vergrößerte Darstellungen der Falten 10 abbilden. Bezüglich gleicher Bezugszeichen kann auf die vorbestehende Figurenbeschreibung verwiesen werden.

Gemäß den Figuren 3 und 4 besteht jede Falte 10 aus zwei sich parallel gegenüberliegenden Faltenwänden 13, die in Körperkontakt einander anliegen bzw. durch einen dünnen Sekretfilm voneinander getrennt sind. Im letzteren Fall bildet der Sekretfilm eine gewisse Haftschicht zwischen den beiden Faltenwänden 13.

Die beiden Faltenwände bilden zusammen einen Faltensteg. Der Faltensteg ist typischerweise von der Anlagefläche 9 der Blase radial nach innen ausgerichtet.

Bei der Faltenbildung entsteht zwischen der Trachea 2 und den nach innen verlaufenden Faltenwänden 13 ein kleiner lichter Zwickel 14, in dem sich geringfügig Sekret ansammelt. Dieser Zwickel 14 gibt auch bei Cuffblasen größerer Wandstärke nur ein kleines Lumen frei, da sich die Faltenwände in diesem Bereich nur um 90 Grad biegen müssen. In vivo verschließt er sich weitgehend durch die weiche Trachealschleimhaut, die bereits bei niedrigem Anpressdruck in ihn hineinprolabiert. Der zum Verschluss dieser Zwickel 14 notwendige Anpressdruck kann bei Verwendung von dünnwandigsten Cuffmaterialien weiter reduziert werden.

Während sich bei Cuffblasen herkömmlicher Wandstärke (ca. 0,06 mm bis 0,1 mm) am Faltengrund eine Faltenöse 12 ausbildet, die flüissigkeitsleitende Kapillaren formiert, bildet sich bei der erfindungsgemäßen Anwendung dünnster Folien zur Cuffblasenkonstruktion ein Faltenumschlag 12 aus, der in seinem Durchmesser so klein ist, dass der freie Durchfluss des Sekretes am Faltengrund gehemmt bzw. ganz unterbunden wird. Der Durchmesser der Faltenöse beträgt vorzugsweise weniger also 0,1 oder 0,05 mm.

In Figur 3 sind bei einer Falte 10 die sich gegenüberliegenden Faltenwände 13 im Bereich des Faltengrundes durch Verschweißen oder Verkleben ummittelbar miteinander verbunden. Somit ist die Faltenöse 12 als Ursache einer möglichen Leckage von Sekreten vermieden. Der an diesem Verbindungsbereich 16 angrenzende Faltenabschnitt 17 wird aus sich gegenüberliegenden Faltenwänden 13 gebildet. Diese sind nicht verbunden und bilden den variablen Faltenabschnitt, wobei sich die Faltentiefe 15 dem jeweiligen trachealen Durchmesser entsprechend einstellt.

Die Faltenöse 12 kann in Kapillargröße ausgebildet sein, bei der die tatsächliche Strömungsgeschwindigkeit des Sekretes geringer ist als die durch den freien Querschnitt der Faltenöse theoretisch ohne Adhäsions- und Viskositätskräfte mögliche Strömungsgeschwindigkeit. Der hemmende Effekt der Adhäsions- und Viskositätskräfte kann bei entsprechender Größe der Faltenöse so ausgeprägt sein, dass die Faltenöse 12 zwar mit Sekret gefüllt, der Durchfluss von Sekreten aber nicht möglich ist.

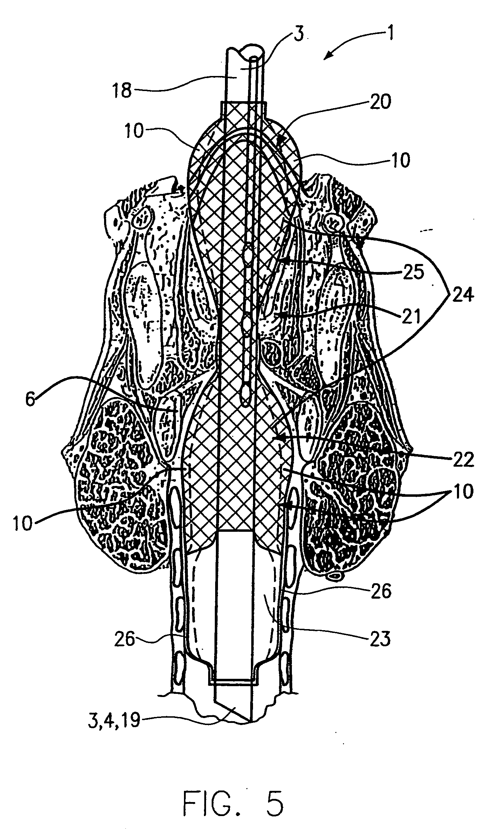

In Figur 5 ist eine zweite Ausführungsform eines Enclotrachealtubus 1 mit den topographischen Beziehungen zu den relevanten angrenzenden anatomischen Strukturen (Kehlkopf und obere Luftröhre) dargestellt. Der Trachealtubus passiert von kranial 18 nach kaudal 19 den Kehldeckel (Epiglottis) 20, die Stimmlippen (Glottis) 21, und den sich an die Stimmlippen nach kaudal anschließenden sog. subglottischen Raum 22, der vom oberen Rand der Cuffmanschette 23 begrenzt ist. Die mechanische Verankerung des Trachealtubus erfolgt unterhalb des Ringknorpels 6, vorzugsweise jedoch im Bereich der mittleren Trachea durch eine luftgeblockte Cuffmanschette 23.

Die Cuffmanschette ist innerhalb einer Tamponierblase 24 angeordnet, wobei Tamponierblase und Cuffmanschette separate Lumen haben, die unabhängig voneinander befüllt werden können. Die Cuffmanschette 23 und Tamponierblase 24 liegen einander im gefüllten Zustand dicht an.

Die Tamponierblase 24 erstreckt sich wahlweise bis in den subglottischen Raum 22, bis zur Glottisebene 21 oder geringfügig darüber hinaus bis in den supraglottischen Bereich 25.

Die Cuffmanschette 23 muss nicht unbedingt innerhalb der Tamponierblase 24 angeordnet sein. Sie kann auch in sequentieller Folge (nach kranial hin) auf dem Tubus angeordnet sein, wobei sie aber vorteilhafterweise in gefülltem Zustand in unmittelbarer, dichter Anlage sein sollen, damit sich in dem Bereich zwischen den Blasen kein Reservoir für Keime bilden kann. Zu diesem Zweck können die Blasen im gemeinsamen Kontaktbereich dauerhaft verbunden sein.

Im Bereich der Auflagefläche 26 der Cuffmanschette 23 an der Tamponierblase 24 sind drei Gestaltungsvarianten möglich. Cuffmanschette 23 und Tamponierblase 24 können nicht miteinander verbunden sein.

Ferner können die beiden Blasen in der gemeinsamen Auflagefläche 26 in einem variablen distalen Abschnitt (z.B. distales Drittel oder distale Hälfte) miteinander verklebt bzw. verschweißt sein. Eine Verklebung bzw. Verschweißung der distalen gemeinsamen Auflagefläche 26 soll verhindern, dass die dünnwandige Tamponierblase nach kaudal hin herniiert, bzw. ihre Trümmer bei einer Ruptur der Tamponierblase nach kaudal umschlagen und am kaudalen Ende 4 der Beatmungskanüle einen ventilartigen Mechanismus formieren.

Schließlich können in funktioneller Analogie zur sequentiellen Anordnung, beide Blasen im Bereich der gesamten gemeinsamen Auflagefläche miteinander verklebt sein.

Die Tamponierblase 24 ist mit einem geeigneten flüssigen oder gasförmigen Fluid gefüllt.

Die zuvor beschriebenen Prinzipien zur dichtenden und schleimhautschonenden Cuffblasengestaltung können auf die Tamponierblase 24 und/oder die fixierende Cuffmanschette 23 angewendet werden.

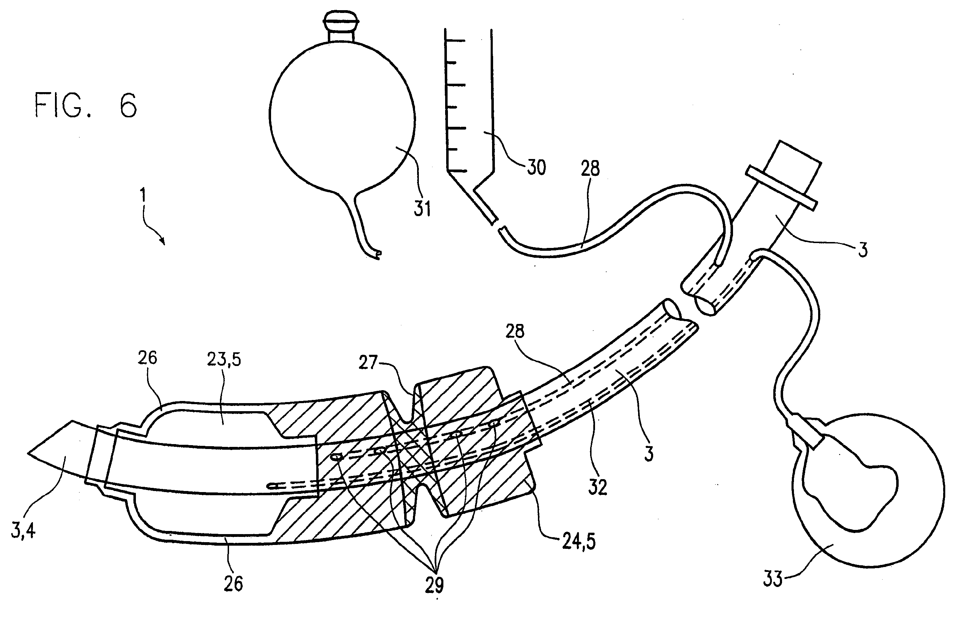

In Figur 6 ist eine dritte Ausführungsform eines erfindungsgemäßen Trachealtubus abgebildet. Der Trachealtubus 1 ist in gefülltem, frei entfaltetem Zustand dargestellt. Im Unterschied zur zweiten Ausführungsform ist in der dritten Ausführungsform die Tamponierblase 24 im Bereich der späteren Stimmlippenplatzierung mit einem präformierten Einschnitt 27 versehen, der in etwa der anatomischen Struktur der Glottis 21 entspricht. Vorzugsweise ist der Einschnitt 27 bei den gewählten Drucken nicht oder nur wenig dehnbar, so dass die Glottis keinem übermäßigen Druck ausgesetzt ist.

In der Beatmungskanüle 3 erstreckt sich ein erster Kanal 28, der über mehrere Austrittsöffnungen 29 mit der Tamponierblase 24 verbunden ist. Der Kanal 28 ist in den Schaft des Tubus integriert und extrakorporal mit einem skalierten Reservoir, bei flüssigen Füllmedien zum Beispiel einer Wassersäule 30, verbunden. Das Reservoir kann jedoch auch als dehnbarer Ausgleichsballon 31 (für flüssige oder gasförmige Medien) gestaltet sein. Der Ausgleichsballon sollte in seinem Volumen mindestens dem gemeinsamen Volumen von Tamponierblase und Cuffmanschette entsprechen.

Das Material des Ausgleichsballons 31 ist vorzugsweise leichter dehnbar als das Material der Tamponierblase 24. Druckanstiege innerhalb der Tamponierblase führen so bevorzugt zu einer Aufdehnung des Ausgleichsballons, eine Aufdehnung der Strukturen des Kehlkopfes kann so weitgehend vermieden werden.

Die Austrittsöffnungen 29 sind in Größe und Anzahl derart gewählt, dass eine rasche Volumenverschiebung zwischen Ausgleichsballon 31 und Tamponierblase 24 möglich ist.

Die Cuffmanschette 23 ist über einen zweiten, in den Tubusschaft integrierten Kanal 32 mit einem Füllballon 33 verbunden. Der Füllballon kann entsprechend dem Lanz'schen Prinzip mit einem selbstregulierenden Ventilmechanismus oder einer einfachen ventiltragenden Reservoirblase ausgestattet sein.

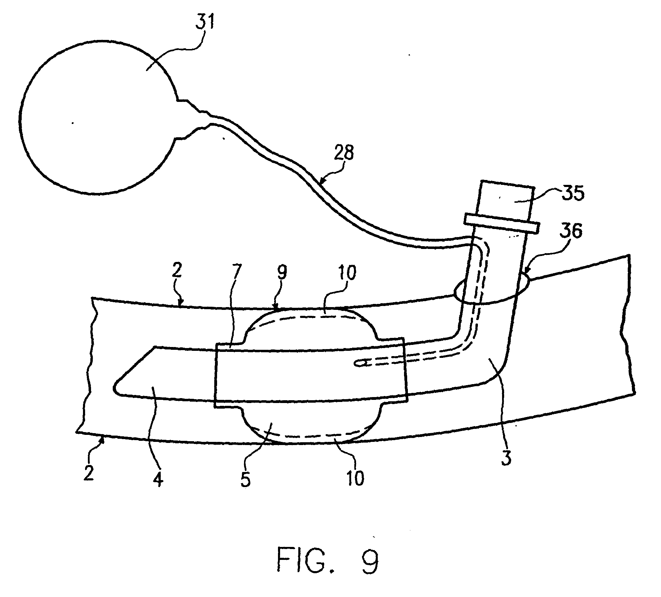

In den Figuren 7 und 8 ist eine besondere Ausführungsform des Ausgleichsballons 31 dargestellt.

In dem in Figur 7 dargestellten Zustand ist der Ausgleichsballon 31 nicht gefüllt, so dass ein auf seiner Wandung aufgedrucktes Muster 34 eine irreguläre Verformung annimmt. In Figur 8 ist der Ballon aufgedehnt, so dass das aufgedruckte Muster 34 in einer geraden regelmäßigen Formgestalt erscheint, welche den optimalen Fü-Ildruck der Tamponierblase 24 anzeigt.

In analoger Weise könnte über eine entsprechende Gestaltveränderung des Ausgleichsballons selbst, vom nicht gefüllten zum gefüllten Zustand, auf den korrekten Fülldruck innerhalb der Tamponierblase geschlossen werden.

Denkbar ist auch eine kompakte, ineinandergeschachtelte Anordnung der der beiden Reservoirblasen zur Versorgung von Cuff- und Tamponierblase in einer gemeinsamen Fassung. Während die mit höherem Fülldruck beaufschlagte innere Blase die Fixier-Cuffmanschette versorgt, hält die äußere Reservoirblase die Tamponierblase mit mäßigem Fülldruck entfaltet.

In Figur 9 ist als vierte Ausführungsform einer erfindungsgemäßen Trachealbeatmungsvorrichtung eine Trachealkanüle 35 dargestellt. Sie wird nicht über die natürlichen Luftwege eingebracht, sondern über ein sog. Stoma 36, welches chirurgisch an der vorderen Halsseite angelegt wird.

Die Beatmungskanüle 3 verläuft durch das Stoma 36 und knickt nach dem Eintritt in die Luftröhre 2 etwa rechtwinklig nach kaudal ab. Im Übrigen ist die Cuffblase 5 analog ausgebildet, wie bezüglich den Figuren 1 bis 4, so dass hinsichtlich gleicher Bezugszeichen auf die vorherigen Ausführungen verwiesen wird. Analog ist der Ausgleichsballon 31 mit dem Kanal 28 so ausgebildet, wie betreffend Figur 6 beschrieben, so dass diesbezüglich auf diese Ausführungen verwiesen wird.

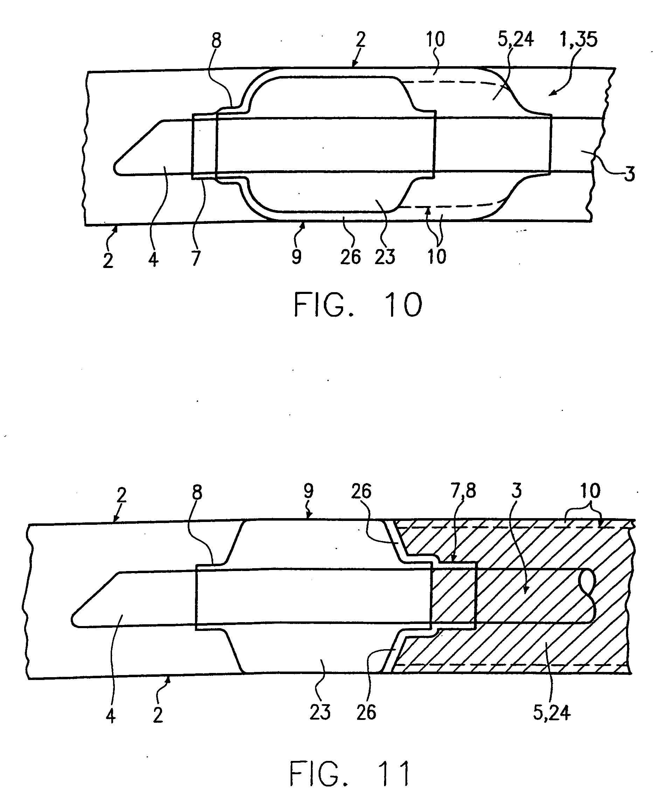

In Figur 10 ist eine fünfte Ausführungsform einer Trachealbeatmungsvorrichtung dargestellt, die als Trachealtubus 1 oder als Trachealkanüle 35 verwendbar ist. Diese Ausführungsform entspricht der in Figur 5 dargestellten Ausführungsform. Gleiche Teile sind mit gleichen Bezugszeichen versehen, so dass diesbezüglich auf die Ausführungen zu Figur 5 verwiesen wird.

Im Unterschied zu Figur 5 ist bei der fünften Ausführungsform in Figur 10 die Tamponierblase 24 kürzer ausgebildet und reicht nicht bis in den Bereich der Glottis hinein. Sie entspricht in ihrer Ausdehnung etwa der fixierenden Cuffmanschette 23. Aus Übersichtlichkeitsgründen sind die beiden separaten Füllkanäle 28 und 29 nicht eingezeichnet.

Nach dem Einbringen der Kanüle wird zur Dichtung primär die äußere Tamponierblase gefüllt. Ist eine zusätzliche mechanische Stabilisierung der Trachealkanüle erforderlich, wird zusätzlich die innere fixierende Cuffmanschette entfaltet. Durch Einbringung eines Trennmittels zwischen die Cuffblasen wird die gegenseitige mechanische Beeinflussung der Cuffmanschette 23 und Tamponierblase 24 verhindert. Zur Gewährleistung der Dichtung der Trachealkanüle wird die Entfaltung der Tamponierblase über ein kommunizierendes, extrakorporal angebrachtes Reservoir mit einem milden Fülldruck von vorzugsweise 10 bis 15 mbar aufrechterhalten.

In Figur 11 ist ausschnittsweise eine sechste Ausführungsform einer erfindungsgemäßen Trachealbeatmungsvorrichtung dargestellt. Diese Ausführungsform ist von der fünften Ausführungsform gemäß Figur 10 entwickelt. Gleiche Teile sind mit gleichen Bezugszeichen versehen, so dass auf die Ausführungen zu Figur 10 verwiesen werden kann.

Im Unterschied zu der fünften Ausführungsform, sind bei der sechsten Ausführungsform die fixierende Cuffmanschette 23 und die Tamponierblase 24 streng sequentiell hintereinander an der Beatmungskanüle 3 angeordnet. An der gemeinsamen Auflagefläche 26 von Cuffmanschette 23 und Tamponierblase 24 liegen ihre Wandungen unmittelbar aneinander an, so dass sich kein Erregerreservoir bilden kann. Wahlweise können die Wandungen im Bereich der Auflagefläche 26 miteinander verklebt oder verschweißt sein.

Im Folgenden wird die Wirkungs- und Funktionsweise der in der Zeichnung dargestellten Ausführungsbeispiele eines erfindungsgemäßen Trachealtubus näher erläutert.

Der Trachealtubus wird bei der Intubation über den Ringknorpel des Kehlkopfes hinweg bis in den Bereich der oberen Trachealspangen vorgeschoben, so dass die Cuffblase 5 sicher im trachealen Bereich zu liegen kommt. Im gefüllten, ohne Begrenzung frei aufgefalteten Zustand ist die Gestalt der Cuffblase größer als im gefüllten Zustand in der Trachea platziert. In der Trachea platziert liegt die Cuffblase 5 mit Faltenwurf an der Anlagefläche 9 der Wand der Trachea an.

Die Cuffblase 5 ist derart gestaltet, dass sich am toten Ende einer Falte 10, eine Faltenöse 12 von derart geringem Durchmesser bildet, dass der Sekretfluss gehemmt wird bzw. ganz zum Erliegen kommt. Dies erfolgt vorzugsweise durch die Viskosität des Sekretes bzw. durch die innerhalb der Kapillaröse auf das Sekret wirkenden Adhäsionskräfte. Die Aspiration von Sekret kann so verhindert und der Entstehung einer beatmungsassoziierten Pneumonie vorgebeugt werden.

Der geringe Durchmesser der Faltenösen 12 kann durch die Verwendung bestimmter, nur wenige Mikrometer starker, flexibler Folien bei der Cuffblasengestaltung erreicht werden.

Ebenfalls können variable Bereiche der sich gegenüberliegenden Faltenwände 13 z.B. durch Verkleben oder Verschweißen miteinander verbunden sein. Idealerweise erfolgt eine derartige Verbindung der Faltenwände 13 im Bereich der Faltenöse 12, so dass jeder Sekretfluss unterbunden ist.

Die erfindungsgemäße Trachealbeatmungsvorrichtung kann auch als Magensonde ver1. wendet werden. Dabei wird die Magensonde anstatt in die Trachea in den Ösophagus eingeschoben, wobei diesbezüglich eine entsprechende Markierung am Sondenschaft zur Positionierung der Sonde vorgesehen ist. Im Ösophagus kann dann die Cuffblase genauso frei aufgefaltet werden, wobei sie mit Faltenwurf an der Wand des Ösophagus spannungsfrei anliegt und am toten Ende der entstehenden Falten Faltenösen mit einem derart geringen Durchmesser ausbildet, der dem freien Durchfluss von Sekreten durch die Faltenösen hemmend entgegenwirken. Dadurch lässt sich eine schonende dem transmuralen Gewebedruck angepasste Austamponierung des Ösophagus erreichen. Das Gewebe der Speiseröhre wird nicht unter Spannung genommen. Das momentan freigegebene Lumen der Speiseröhre wird unter Erhalt der Ösophagusfaltung lediglich austamponiert. Die Magensonde dichtet so auf langzeitvertragliche Weise gegen aufsteigendes gastrointestinales Sekret.

Obwohl Magensonden und Trachealbeatmungsvorrichtung bisher stets als separate Geräte konstruiert wurden und gegenseitig für die jeweils andere Verwendung nicht austauschbar waren, ist mit der erfindungsgemäßen Vorrichtung sowohl eine Anwendung als Trachealbeatmungsvorrichtung als auch als Magensonde möglich. Die Beatmungskanüle wird im Fall der Magensonde als inneres kanalartiges Lumen verwendet, durch welches eine eigentliche Emährungssonde durchgeführt werden kann oder über das selbst die Ernährungsversorgung erfolgt.

Die Magensonde kann auch im Sinne einer sogenannten Senstaken-Blakemore-Sonde verwendet werden. In diesem Fall wird die Cuffblase ebenfalls im Ösophagus platziert. Damit ist es möglich, die Durchblutung der umliegenden Gefäße der Speiseröhre durch Variation des Fülldruckes der Cuffblase zu beeinflussen. Zum Beispiel können Blutungen umliegender Schleimhaut-Gefäße gestoppt werden. Die Ausbildung der spezifischen, zuvor beschriebenen Entfaltung des residual gestalteten Cuffmantels, gestattet es hier ebenfalls, die Cuff-Füllung dem nötigen Gefäßverschlugdruck exakt anzupassen ohne dabei den Cuffmantel und daran angrenzende Strukturen unter nennenswerte Spannung zu nehmen. Gefäßblutungen können so mit minimal nötigem Druck unterbunden werden. Die Schleimhaut des Ösophagus wird so geringst möglich traumatisiert. Bei der Verwendung als Sengstake dient das innere kanalartige Lumen als Sondenschaft. Das magenseitige Ende des Sondenschafts kann offen oder geschlossen gestaltet sein.

## Patentansprüche

1. Trachealbeatmungsvorrichtung (1, 35), insbesondere Trachealtubus oder Trachealkanüle, die die Trachea (2) zum Beatmen eines Patienten möglichst dicht verschließt mit einer die Trachea (2) unterhalb der Glottis (21) blockierenden Cuffblase (5), durch die eine Beatmungskanüle (3) hindurchgeführt ist, wobei die Cuffblase (5) in gefülltem, ohne Begrenzung frei auffaltbarem Zustand größer ist als in gefülltem Zustand in der Trache (2) platziert, und aus flexiblem Weichfolienmaterial besteht, wobei die Cuffblase (5) mit Faltenwurf (10) an der Trachea (2) anlegbar ist,
**dadurch gekennzeichnet,**
**dass** die am toten Ende (11) einer Falte (10) entstehende Faltenöse (12) einen Durchmesser von weniger als 0,1 mm aufweist, der dem freien Durchfluß des Sekrets durch die Faltenöse (12) hemmend entgegenwirkt.

2. Trachealbeatmungsvorrichtung (1. 35) nach Anspruch 1,
**dadurch gekennzeichnet,**
**dass** der Durchmesser der Faltenöse (12) weniger als 0,05 mm beträgt.

3. Trachealbeatmungsvorrichtung (1, 35) nach Anspruch 1 oder 2,
**dadurch gekennzeichnet,**
**dass** die Faltenöse (12) in Kapillargröße ausgebildet ist, bei der die tatsächliche Strömungsgeschwindigkeit des Sekrets geringer ist als die durch den freien Querschnitt theoretisch ohne Adhäsions-Viskositätskräfte mögliche Strömungsgeschwindigkeit.

4. Trachealbeatmungsvorrichtung (1, 35) nach einem der v orhergehenden Ansprüche,
**dadurch gekennzeichnet,**
**dass** das Folienmaterial der Cuffmanschette (5) aus Polyethylenteraphtalat (PETP), niedrig dichtem Polyethylen (LDPE), Polyvinylchlorid (PVC) oder Polyurethan (PU) besteht.

5. Trachealbeatmungsvorrichtung (1, 35) nach einem der vorhergehenden Ansprüche,
**dadurch gekennzeichnet,**
**dass** die Wanddicke der Folie im Bereich des Faltenwurfes (10) dünner ist als im an der Trachealschleimhaut unmittelbar anliegende faltenfreien Bereich (9).

6. Trachealbeatmungsvorrichtung (1, 35) nach einem der vorhergehenden Ansprüche,
**dadurch gekennzeichnet,**
**dass** die sich in einer Falte (10) gegenüberliegenden Faltenwände (13) im Bereich nahe des Faltengrundes (111) miteinander verbunden sind.

7. Trachealbeatmungsvorrichtung (1, 35) nach Anspruch 6,
**dadurch gekennzeichnet,**
**dass** die sich gegenüberliegenden Faltenwände (13) am toten Ende (11) der Falte (11) direkt im Faltengrund (11) die Faltenöse (12) ausfüllend miteinander verbunden sind.

8. Trachealbeatmungsvorrichtung (1, 35) nach Anspruch 6 oder 7,
**dadurch gekennzeichnet,**
**dass** die sich gegenüberliegenden Faltenwände (13) einer Falte (10) miteinander verschweißt oder verklebt sind.

9. Trachealbeatmungsvorrichtung (1, 35) nach einem der Ansprüche 6 bis 8,
**dadurch gekennzeichnet,**
**dass** sich in der Falte (10) an den Verbindungsbereich (16) der sich gegenüberliegenden Faltenwände (13) angrenzend ein im Querschnitt in der Faltentiefe (15) variabler Faltenabschnitt (17) anschließt, in dem die sich gegenüberliegenden Faltenwände (13) nicht stofflich miteinander verbunden sind.

10. Trachealbeatmungsvorrichtung (1, 35) nach einem der vorhergehenden Ansprüche,
**dadurch gekennzeichnet,**
**dass** die Cuffblase (5) aus einem bei Faltenwurf (10) leicht an sich selbst anhaftenden Material besteht.

11. Trachealbeatmungsvorrichtung (1, 35) nach einem der vorhergehenden Ansprüche,
**dadurch gekennzeichnet,**
**dass** eine als Cuffblase ausgebildete Tamponierblase (24) und eine unterhalb der Glottis (21) positionierbare separate Fixier-Cuffmanschette (23) an der Beatmungskanülr (3) vorgesehen ist.

12. Trachealbeatmungsvorrichtung (1, 35) nach Anspruch 11,
**dadurch gekennzeichnet,**
**dass** die Fixier-Cuffmanschette (23) an der kaudalen Seite der Vorrichtung und die Tamponierblase (24) relativ dazu cranial vorgesehen ist.

13. Trachealbeatmungsvorrichtung (1, 35) nach Anspruch 11 oder 12,
**dadurch gekennzeichnet,**
**dass** die Fixier-Cuffmanschette (23) und die Tamponierblase (24) etwa sequentiell hintereinander längs der Beatmungskanüle (3) angeordnet sind.

14. Trachealbeatmungsvorrichtung (1, 35) nach einem der Ansprüche 11 bis 13,
**dadurch gekennzeichnet,**
**dass** die Fixier-Cuffmanschette (23) in gefülltem Zustand unmittelbar an der Tamponierblase (24) an einer gemeinsamen Auflagefläche (26) anliegt.

15. Trachealbeatmungsvorrichtung (1. 35) nach einem der Ansprüche 11 bis 14,
**dadurch gekennzeichnet,**
**dass** die Fixier-Cuffmanschette (23) mindestens bereichsweise, vorzugsweise vollständig, von der Tamponierblase (24) umschlossen ist.

16. Trachealbeatmungsvorrichtung (1, 35) nach einem der Ansprüche 11 bis 15,
**dadurch gekennzeichnet,**
**dass** zwischen Fixier-Cuffmanschette (23) und Tamponierblase (24) ein Gleitmittel vorgesehen ist.

17. Trachealbeatmungsvorrichtung (1, 35) nach Anspruch 16,
**dadurch gekennzeichnet,**
**dass** das Gleitmittel an der in gefülltem Zustand gemeinsamen Auflagefläche (26) von Tamponierblase (24) und Fixier-Cuffmanschette (23) vorgesehen ist.

18. Trachealbeatmungsvorrichtung (1, 35) nach einem der Ansprüche 11 bis 17,
**dadurch gekennzeichnet,**
**dass** Tamponierblase (24) und Fixier-Cuffmanschette (23) separat voneinander befüllbar sind.

19. Trachealbeatmungsvorrichtung (1, 35) nach einem der Ansprüche 11 bis 18,
**dadurch gekennzeichnet,**
**dass** die Tamponierblase (24) in ihrer Größe in gefülltem Zustand zum Ausfüllen des subglottischen Raumes (22) ausgebildet ist.

20. Trachealbeatmungsvorrichtung (1, 35) nach einem der Ansprüche 11 bis 19,
**dadurch gekennzeichnet,**
**dass** die Tamponierblase (24) derart ausgebildet ist, dass sie sowohl den subglottischen (22) als auch den supraglottischen (25) Raum ausfüllt.

21. Trachealbeatmungsvorrichtung (1, 35) nach einem der Ansprüche 11 bis 20,
**dadurch gekennzeichnet,**
**dass** die Gestalt der Tamponierblase (24) in gefülltem Zustand verschieden von der Gestalt der Cuffmanschette (23) ist.

22. Trachealbeatmungsvorrichtung (1, 35) nach einem der Ansprüche 11 bis 21,
**dadurch gekennzeichnet,**
**dass** die Foliendicke der Fixier-Cuffmanschette (23) größer ist als die der Tamponierblase (24).
